# FASCICULE DE BREVET EUROPEEN

(11) **EP 2 055 200 B1**
(45) Date de publication et mention de la délivrance du brevet: **12.01.2011**
(21) Numéro de dépôt: 08167893.0
(22) Date de dépôt: 30.10.2008
(51) Int. Cl.: A23L 1/29

(54) **Lot de compléments alimentaires comprenant l'association d'un extrait d'une plante appartenant à la famille des rosacées et un extrait de Criste-Marine.**
Charge von Nahrungsergänzungsmitteln, die eine Mischung aus einem Pflanzenextrakt einer zur Gattung der Rosaceen gehörenden Pflanze und einem Extrakt von Criste Marine (Meerfenchel) enthält
Batch of food supplements comprising a blend of an extract of a plant belonging to the rosaceous family and an extract of sea fennel.

(30) Priorité: 31.10.2007 FR 0707693
(43) Date de publication de la demande: 06.05.2009
(73) Titulaire: Institut Europeen de Gestion de la Sante (IEGS), 22740 Pleudaniel (FR)
(72) Inventeur: Dubreuil, Jacques, 22000 Saint Brieuc (FR)
(74) Mandataire: Maillet, Alain

(56) Documents cités:
- FR-A- 2 650 941
- BIODETENTE.COM: "Christe Marine 90 gélules végétales" ARTICLE INTERNET, [Online] 14 mars 2006 (2006-03-14), XP002488545 Extrait de l'Internet: URL:http://www.biodetente.com/christe-mari ne-gelules-vegetales-p-38.html> [extrait le 2008-07-16]
- BOUTIQUE-NATURE.FR: "MENO?MINCE" ARTICLE INTERNET, [Online] 3 septembre 2006 (2006-09-03), XP002488546 Extrait de l'Internet: URL:http://web.archive.org/web/20060903082 853/http://www.boutique-nature.fr/fiche_pr oduit.php?id=106> [extrait le 2008-07-16]
- ARTEMIS HERBS: "Lady's mantle herb" ARTICLE INTERNET, [Online] 2005, XP002488547 Extrait de l'Internet: URL:http://www.artemisherbs.co.uk/artemis/ c_viewnotes.jsp?hl=true&id=T10075-100> [extrait le 2008-07-16]
- MYBEAUTIFULBELLY.NET: "Femmune Capsules 330mg" ARTICLE INTERNET, [Online] 12 avril 2007 (2007-04-12), XP002488548 Extrait de l'Internet: URL:http://mybeautifulbelly.net/community/ index.php?option=com_content&task=view&id= 94&Itemid=47> [extrait le 2008-07-16]

## Description

La présente invention concerne un complément alimentaire comprenant au moins un extrait d'une plante appartenant à la famille des rosacées, en particulier la potentille quintefeuille, la potentille ansérine ou l'alchémille, et un extrait de Criste-Marine. La présente invention concerne également un lot de compléments alimentaires comprenant au moins un extrait d'une plante appartenant à la famille des rosacées, en particulier la potentille quintefeuille, la potentille ansérine ou l'alchémille, et un extrait de Criste-Marine.

La Criste-Marine, *Crithmum maritimum L,* est une plante de la famille des Apiaceaes que l'on trouve sur les falaises et les littoraux rocheux ou sablonneux de l'ouest de l'Europe. Utilisée depuis la nuit des temps pour ses propriétés antiscorbutique, digestive et dépurative, elle est également très répandue en cosmétologie en tant qu'ingrédient de crèmes ou de compléments alimentaires aux vertus raffermissantes ou amincissantes. Elle est également commercialisée sous forme de complément alimentaire pour ses vertus reconstituantes et stimulantes.

Les plantes de la famille des rosacées, telles que la potentille quintefeuille, la potentille ansérine ou l'alchémille, sont également connues pour leurs vertus en matière de santé humaine. La potentille quintefeuille, *potentilla reptans L,* est connue pour ses propriétés astringentes, toniques et digestives. La potentille ansérine, *potentilla anserina*, est également connue pour ses propriétés astringentes et est efficace contre les maux de gorge et les diarrhées. L'alchémille, *Alchemillae vulgaris*, est utilisée pour lutter contre les diarrhées et atténuer les syndromes prémenstruels.

Les demandeurs ont découvert, de façon surprenante, que l'association de la Criste-Marine avec une plante choisie parmi la famille des rosacées permettait d'obtenir un effet de synergie se manifestant par une amélioration de l'état de santé général de sujets souffrants de maladies diverses.

L'invention concerne ainsi un complément alimentaire comprenant l'association d'au moins un extrait d'une plante appartenant à la famille des rosacées, en particulier la potentille quintefeuille, la potentille ansérine ou l'alchémille, et d'un extrait de Criste-Marine.

Selon un mode de réalisation de l'invention, ladite plante appartenant à la famille des rosacées est la potentille ansérine. Les demandeurs ont observé que l'association d'un extrait de potentille ansérine et d'un extrait de Criste-Marine permettait une amélioration de l'état de santé général de personnes souffrant de troubles dus au vieillissement.

Selon un autre mode de réalisation de l'invention, ladite plante appartenant à la famille des rosacées est la potentille quintefeuille. Les demandeurs ont remarqué que l'association des ces deux extraits de plantes permettaient de diminuer les symptômes liés aux maladies inflammatoires ou aux maladies auto-immunes et de diminuer les risques de rejets de greffons.

Selon encore un autre mode de réalisation de l'invention, ladite plante appartenant à la famille des rosacées est l'alchémille. Les demandeurs ont pu observer que l'association d'un extrait d'alchémille et d'un extrait de Criste-Marine aidait à maintenir la condition physique des patients en cours de traitement contre un cancer.

La présente invention concerne également un lot de compléments alimentaires comprenant au moins un complément alimentaire comprenant un extrait d'une plante appartenant à la famille des rosacées, en particulier la potentille quintefeuille, la potentille ansérine ou l'alchémille, et un complément alimentaire comprenant un extrait de Criste-Marine. Les extraits des plantes peuvent être séparés physiquement mais réunis au sein d'un même lot de compléments alimentaires, de manière à être pris conjointement par l'utilisateur.

Selon un mode de réalisation particulièrement avantageux de l'invention, l'extrait de la plante de la famille des rosacées est un extrait conservé en milieu hydro alcoolique. Les demandeurs ont en effet observé que l'extraction en milieu hydro alcoolique permettait la récupération d'une grande quantité de principes actifs des plantes appartenant à la famille des rosacées et que ces principes actifs étaient mieux conservés en milieu hydro alcoolique que sous une forme sèche. Les procédés d'extractions utilisés sont ceux bien connus de l'homme de l'art.

Selon le même mode de réalisation de l'invention, l'extrait de Criste-Marine est un extrait sec sous forme de poudre.

L'invention concerne encore l'utilisation d'au moins un extrait d'une plante appartenant à la famille des rosacées et d'un extrait de Criste-Marine pour la fabrication d'un médicament.

L'invention sera mieux comprise à la lecture des exemples de réalisation suivants, qui se veulent illustratifs et non limitatifs.

### Exemple 1 : Lot de compléments alimentaires comprenant un extrait de Potentille Quintefeuille et un extrait de Criste-Marine :

Le lot de compléments alimentaires comprend :
- un flacon comprenant 120 gélules d'un extrait sec sous forme de poudre de Criste-Marine. Chaque gélule comprend 330mg d'extrait sec. L'extrait sec peut être obtenu par tout procédé d'extraction connu de l'homme du métier.
- un flacon muni d'un compte gouttes comprenant 125 mL d'un extrait hydro alcoolique de potentille quintefeuille. L'extrait hydro alcoolique peut être obtenu à partir des parties aériennes de la plante par tout procédé d'extraction en eau et alcool connu de l'homme du métier et se présente sous une forme liquide comprenant, au final, 45 % d'alcool, en volume.

Le dosage optimal par jour est de 60 gouttes d'extrait hydro alcoolique de potentille quintefeuille diluées dans un verre d'eau et associées à la prise de 2 à 6 gélules d'extrait sec de Criste-Marine.

### Exemple 2 : Lot de compléments alimentaires comprenant un extrait de Potentille Ansérine et un extrait de Criste-Marine :

Le lot de compléments alimentaires comprend :
- un flacon comprenant 120 gélules d'un extrait sec sous forme de poudre de Criste-Marine. Chaque gélule comprend 330mg d'extrait sec. L'extrait sec peut être obtenu par tout procédé d'extraction connu de l'homme du métier.
- un flacon comprenant 125 mL d'un extrait hydro alcoolique de potentille ansérine. L'extrait hydro alcoolique peut être obtenu à partir des parties aériennes de la plante par tout procédé d'extraction en eau et alcool connu de l'homme du métier et se présente sous une forme liquide comprenant, au final, 45 % d'alcool en volume.

Le dosage optimal par jour est de 60 gouttes d'extrait hydro alcoolique de potentille ansérine diluées dans un verre d'eau et associées à la prise de 2 à 6 gélules d'extrait sec de Criste-Marine.

### Exemple 3 : Lot de compléments alimentaires comprenant un extrait d'alchémille et un extrait de Criste-Marine :

Le lot de compléments alimentaires comprend :
- un flacon comprenant 120 gélules d'un extrait sec sous forme de poudre de Criste-Marine. Chaque gélule comprend 330mg d'extrait sec. L'extrait sec peut être obtenu par tout procédé d'extraction connu de l'homme du métier.
- un flacon comprenant 125 mL d'un extrait hydro alcoolique d'alchémille. L'extrait hydro alcoolique peut être obtenu à partir des parties aériennes de la plante par tout procédé d'extraction en eau et alcool connu de l'homme du métier et se présente sous une forme liquide comprenant, au final, 45 % d'alcool en volume.

Le dosage optimal par jour est de 60 gouttes d'extrait hydro alcoolique de potentille ansérine diluées dans un verre d'eau et associées à la prise de 2 à 6 gélules d'extrait sec de Criste-Marine.

## Revendications

1. Complément alimentaire, **caractérisé en ce qu'**il comprend l'association d'au moins un extrait d'une plante appartenant à la famille des rosacées, en particulier la potentille quintefeuille, la potentille ansérine ou l'alchémille, et d'un extrait de Criste-Marine.

2. Complément alimentaire selon la revendication 1, **caractérisé en ce que** ladite plante appartenant à la famille des rosacées est la potentille ansérine.

3. Complément alimentaire selon la revendication 1, **caractérisé en ce que** ladite plante appartenant à la famille des rosacées est la potentille quintefeuille.

4. Complément alimentaire selon la revendication 1, **caractérisé en ce que** ladite plante appartenant à la famille des rosacées est l'alchémille.

5. Lot de compléments alimentaires, **caractérisé en qu'**il comprend au moins un complément alimentaire comprenant un extrait d'une plante appartenant à la famille des rosacées, en particulier la potentille quintefeuille, la potentille ansérine ou l'alchémille, et un complément alimentaire comprenant un extrait de Criste-Marine.

6. Lot de compléments alimentaires selon la revendication 5, **caractérisé en ce que** ledit extrait de la plante de la famille des rosacées est un extrait liquide hydro alcoolique.

7. Lot de compléments alimentaires selon l'une des revendications 5 à 6, **caractérisé en que** ledit extrait de Criste-Marine est un extrait sec sous forme de poudre.

## Claims

1. Food supplement, **characterised in that** it comprises a combination of at least one extract of a plant belonging to the rosaceous family, in particular Potentilla reptans, Potentilla anserina or Alchemilla, and an extract of sea fennel.

2. Food supplement according to claim 1, **characterised in that** said plant belonging to the rosaceous family is Potentilla anserina.

3. Food supplement according to claim I, **characterised in that** said plant belonging to the rosaceous family is Potentilla reptans.

4. Food supplement according to claim 1, **characterised in that** said plant belonging to the rosaceous family is Potentilla Alchemilla.

5. Batch of food supplements, **characterised in that** it comprises at least one food supplement comprising an extract of a plant belonging to the rosaceous family, in particular Potentilla reptans, Potentilla anserina or Alchemilla, and a food supplement comprising an extract of sea fennel.

6. Batch of food supplements according to claim 5, **characterised in that** said extract of the plant belonging to the rosaceous family is a liquid hydroalcoholic extract.

7. Batch of food supplements according to one of claims 5 to 6, **characterised in that** said extract of sea fennel is a dry extract in the form of a powder.

## Patentansprüche

1. Nahrungsergänzungsmittel,
**dadurch gekennzeichnet, dass**
es eine Verbindung von mindestens einem Extrakt aus einer zur Familie der Rosaceen gehörenden Pflanze, insbesondere Fünffingerkraut, Gänsefingerkraut oder Frauenmantel und einen Extrakt aus Meerfenchel umfasst.

2. Nahrungsergänzungsmittel nach Anspruch 1,
**dadurch gekennzeichnet, dass**
die zur Familie der Rosaceen gehörende Pflanze Gänsefingerkraut ist.

3. Nahrungsergänzungsmittel nach Anspruch 1,
**dadurch gekennzeichnet, dass**
die zur Familie der Rosaceen gehörende Pflanze Fünffingerkraut ist.

4. Nahrungsergänzungsmittel nach Anspruch 1,
**dadurch gekennzeichnet, dass**
die zur Familie der Rosaceen gehörende Pflanze Frauenmantel ist.

5. Charge von Nahrungsergänzungsmitteln,
**dadurch gekennzeichnet, dass**
sie mindestens ein, einen Extrakt aus einer zur Familie der Rosaceen, insbesondere Fünffingerkraut, Gänsefingerkraut oder Frauenmantel, gehörenden Pflanze umfassendes Nahrungsergänzungsmittel und ein einen Meerfenchel-Extrakt umfassendes Nahrungsergänzungsmittel umfasst.

6. Charge von Nahrungsergänzungsmitteln nach Anspruch 5,
**dadurch gekennzeichnet, dass**
der Extrakt aus der zur Familie der Rosaceen gehörenden Pflanze ein wässrig-alkoholischer Flüssigextrakt ist.

7. Charge von Nahrungsergänzungsmitteln nach einem der Ansprüche 5 bis 6,
**dadurch gekennzeichnet, dass**
der Meerfenchel-Extrakt ein Trockenextrakt in Pulverform ist.
